# EUROPEAN PATENT APPLICATION

(11) **EP 3 689 228 A1**
(43) Date of publication of application: **05.08.2020**
(21) Application number: 18930409.0
(22) Date of filing: 24.08.2018
(51) Int. Cl.: A61B 5/00, A61B 5/1455

(54) **BIO-SIGNAL ANALYSIS APPARATUS USING MACHINE LEARNING AND METHOD THEREFOR**

(30) Priority: 17.08.2018 KR 20180095928
(71) Applicant: Olive Healthcare Inc., Seoul 05836 (KR)
(72) Inventor: HAN, Sung Ho, Seoul 06649 (KR); HONG, Hee Sun, Seoul 06762 (KR); JEONG, Chang Ki, Seoul 08790 (KR)
(74) Representative: Manitz Finsterwald Patent- und Rechtsanwaltspartnerschaft mbB
(86) International application number: PCT/KR2018/009808
(87) International publication number: WO 2020/036246

(57) **Abstract**

A bio-signal analysis method according to an aspect of the present invention uses a bio-signal analysis apparatus for analyzing a bio-signal based on output light emitted from an object after being output from a light source, and includes a step of providing a classification unit machine-trained based on training data in which measurement values for each wavelength of the output light and concentration for each of a plurality of chromophore materials match each other; a step of inputting the measurement values of the output light emitted from the object measured by the bio-signal analysis apparatus into the classification unit; and a step of outputting chromophore concentration output from the classification unit with respect to the input. At this time, the light source includes a plurality of light emitting elements for emitting output light having different wavelengths.

## Description

### [Technical Field]

The present invention relates to a bio-signal analysis apparatus using machine learning and a method therefor.

### [Background]

Recently, various technologies for analyzing biological information of the body by using optical characteristics of a turbid medium have been developed. The technologies are spotlighted in that the technologies may provide non-invasive and reliable biological information to the body, and a lot of attention is focused on research and development of devices to be widely used according to needs of consumers.

Particularly, many technologies analyze the biological information of the body by measuring an absorption coefficient and a scattering coefficient of a turbid medium in a near infrared region to calculate concentration of chromophore contained in the turbid medium. In general, three methods are known as methods for measuring the absorption and scattering coefficients of the turbid medium. Specifically, there are a steady-state (SS) method for calculating the concentration of chromophore according to a multi-range measurement method after light with a certain intensity is incident on the turbid medium, a frequency-domain (FD) method for measuring changed amplitude and phase with respect to a modulated light source, a time domain (TD) method for measuring a change over time for a light source having a pulse shape, and the like.

Meanwhile, the bio-signal analysis apparatus calculates the concentration of chromophore concentration by generating a plurality of pieces of output light having different wavelengths by using an LD, a VCSEL or an LED as a light source, irradiating an object with the output light, and using measurement values for light emitted from the object. To this end, a calculation model that shows a relationship between the concentration of each chromophore in the object and characteristics of the output light such as an amplitude or a phase of the output light generated according thereto is used, and a fitting algorithm such as a least square method is performed based on the model, and thus, concentration of desirable chromophore is found out.

However, in a case of some light sources, there is a problem that a concentration value of the chromophore calculated through the above-described calculation model and numerical fitting does not match an actual result due to characteristics that a spectral width of the wavelength is wide.

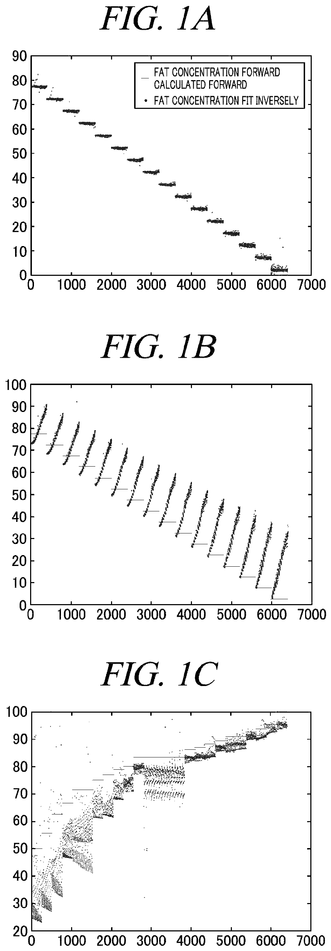

FIGS. 1A, 1B and 1C illustrate diagrams for describing calculation results when a model for calculating the concentration of chromophore is applied to output light according to a general optical element type.

Fig. 1A and Fig. 1B illustrate concentration of fat in the chromophore and are graphs illustrated by using a total of 6,400 pieces of simulation data. An X axis represents an identification number of each piece of experimental data, and an Y axis represents the concentration of chromophore.

Fig. 1A illustrates results of using a VCSEL device, and it may be confirmed that concentration values (orange color) of fat obtained reversely again matches correctly by fitting simulation data for a concentration value (blue color) of fat in each case and VCSEL output light in the 6,400 pieces of data used in the simulation by using a calculation model.

However, Fig. 1B illustrates a case of the LED, and it may be confirmed that the concentration value (blue color) of fat originally set in the simulation does not correctly match the concentration value (orange color) of fat fitted with the simulation data for the LED output light because the LED has a larger width of spectrum of wavelength than the VCSEL.

In addition, Fig. 1C illustrates a simulation result for a VCSEL device and illustrates a result obtained by fitting concentration of hemoglobin rather than fat, and the same problem that prediction based on a current calculation model is not correct occurs even in the VCSEL device due to unique absorption characteristics of hemoglobin, in a case of chromophore such as the hemoglobin.

In order to solve this problem, it is intended to propose a new method for analyzing a bio-signal without performing a fitting process of using a calculation model illustrating a correlation between characteristics of output light and concentration of each chromophore.

### [Disclosure]

### [Technical Problem]

An object of an embodiment of the present invention is to provide a bio-signal analysis apparatus and an operation method of the apparatus for performing bio-signal analysis by using a machine learning method without performing a fitting process of using a calculation model.

However, an object to be achieved by the present embodiment is not limited to the objective described above, and other objectives may exist.

### [Technical Solution]

As technical means for achieving the above-described objectives, a bio-signal analysis method according to an aspect of the present invention uses a bio-signal analysis apparatus for analyzing a bio-signal based on output light emitted from an object after being output from a light source, and includes a step of providing a classification unit machine-trained based on training data in which measurement values for each wavelength of the output light and concentration for each of a plurality of chromophore materials match each other; a step of inputting the measurement values of the output light emitted from the object measured by the bio-signal analysis apparatus into the classification unit; and a step of outputting chromophore concentration output from the classification unit with respect to the input. At this time, the light source includes a plurality of light emitting elements for emitting output light having different wavelengths.

In addition, a bio-signal analysis apparatus according to another aspect of the present invention includes a plurality of light emitting elements that emit output light having different wavelengths to an object; at least one photodetector that detects the output light emitted from the object; and a control unit that is connected to the light emitting element and the photodetector to control operations of the light emitting element and the photodetector and calculates chromophore concentration based on a measurement value of an optical signal received through the photodetector. At this time, the control unit includes a classification unit machine-trained based on training data in which measurement values for each wavelength of the output light and concentration for each of a plurality of chromophore materials match each other, and inputs the measurement values of the output light emitted from the object measured by the photodetector to the classification unit to calculate the chromophore concentration.

### [Advantageous effects]

According to an embodiment of the present invention, a bio-signal analysis apparatus capable of providing classification results of the same performance as a VCSEL element may be implemented even when a relatively inexpensive LED element compared to a VCSEL element is used. In addition, even when the VCSEL device is used, improved classification performance for various chromophore materials may be provided.

### [Description of Drawings]

FIGS. 1A, 1B and 1C illustrate diagrams for explaining how well fat concentration values match each other, the fat concentration values being obtained by performing fitting based on a calculation model by using a true value of fat concentration used in simulation for each general optical element and simulation data.
FIG. 2 is a view illustrating a configuration of a bio-signal analysis apparatus according to an embodiment of the present invention.
FIG. 3 is a graph 200 illustrating an absorption spectrum of chromophores existing in a body.
FIGS. 4A, 4B are diagrams illustrating optical characteristics of input light incident on an object by a light emitting element and output light detected by a photodetector.
FIG. 5 is a diagram illustrating a detailed configuration of a control unit according to an embodiment of the present invention.
FIG. 6 is a diagram illustrating an example of training data used for learning a classification unit according to an embodiment of the present invention.
FIGS. 7A, 7B, 8A, 8B, 9A and 9B are diagrams illustrating analysis results of the bio-signal analysis apparatus according to the embodiment of the present invention.

### [Best Mode]

Hereinafter, an embodiment of the present invention will be described in detail with reference to the accompanying drawings such that those skilled in the art to which the present invention belongs may easily perform. The present invention may, however, be embodied in many different forms and is not limited to the embodiment described herein. In order to clearly illustrate the present invention, parts not related to the description are omitted in the drawings, and similar parts are denoted by similar reference numerals or symbols throughout the specification.

In addition, while referring to the drawings, even in a configuration indicated by the same name, drawing number may change depending on drawings, drawing numbers are simply described for the sake of convenient explanation, and concepts, characteristics, functions, or effects of respective configurations are not limitedly interpreted by the corresponding drawing numbers.

Throughout the specification, when a part is referred to as being "connected" to another part, this includes not only "directly connected" but also "electrically connected" with another element therebetween. In addition, when a part is referred to as "including" a configuration element, it means that the configuration element does not exclude other configuration elements but may further include other configuration elements unless describes otherwise in particular, and it is to be understood that the configuration element does not preclude presence or addition of one or more other features, numerals, steps, operations, configuration elements, components, or a combination thereof.

Throughout the specification, an "object" is a measurement target of a bio-signal analysis apparatus and may include a person, an animal, or a part thereof. In addition, the object may include various organs such as the heart, brain, or blood vessels, or various types of phantoms.

Hereinafter, an embodiment of the present invention will be described in detail with reference to the accompanying drawings.

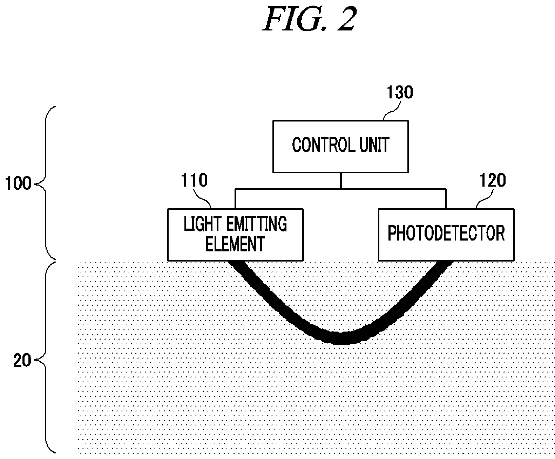

FIG. 2 is a diagram illustrating a configuration of a bio-signal analysis apparatus according to an embodiment of the present invention.

Referring to FIG. 2, the bio-signal analysis apparatus 100 according to the embodiment of the present invention includes a plurality of light emitting elements 110, at least one photodetector 120, and a control unit 130.

At this time, a laser diode (LD), a light emitting diode (LED), or a vertical cavity surface emitting laser (VCSEL) may be used as the light emitting element 110. Each light emitting element 110 may emit output light having different wavelengths. Particularly, the light emitting element 110 may emit output light having a discrete wavelength. Here, the discrete wavelength means a discontinuous wavelength and may be a wavelength in a near infrared ray region. For example, eight light emitting elements 110 may be arranged to output eight wavelengths, and at this time, may emit in discrete wavelengths in a region of 650 to 1,100 nm (nano-meter). The number of the wavelengths may be changed depending on selection, but it is preferable that the number of wavelengths is equal to or greater than the number of chromophore materials.

Each light emitting element 110 may emit light having different discrete wavelengths to an object 20 under a control of the control unit 130. At this time, the discrete wavelength emitted by each light emitting element 110 may be determined based on chromophore existing in the object 20, and specifically, may be determined based on the known light absorbance of each chromophore. Here, the chromophore is an organic compound having an unsaturated bond and means an atom or an atomic group that absorbs light. In general, the type of chromophores existing in a body is restrictive and known. For example, water (H₂O), lipid, oxy-hemoglobin (O₂Hb), and deoxy-hemoglobin (HHb) dominantly exist in tissues such as the arms and legs, and water, oxy-hemoglobin, and deoxy-hemoglobin except lipid dominantly exist in the brain.

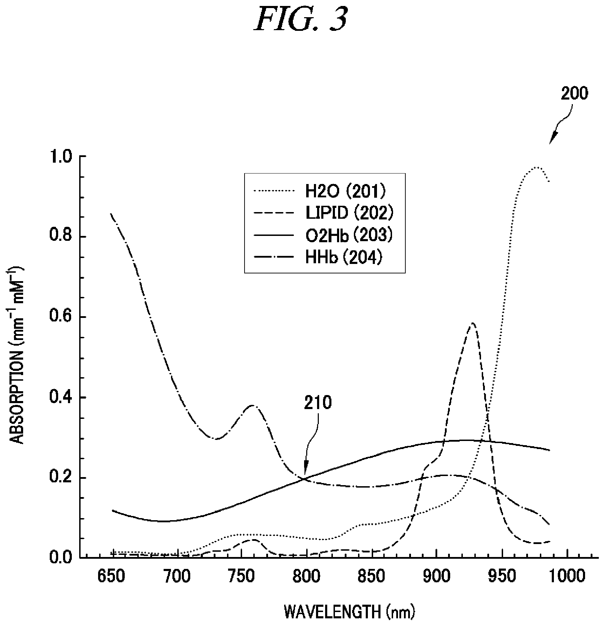

FIG. 3 is a graph 200 illustrating an absorption spectrum of chromophores existing in the body. In general, the chromophores have different absorption spectral characteristics in the near infrared region.

As illustrated in FIG. 3, water 201 has a peak characteristic in a wavelength region of approximately 980 nm, and lipid 202 has a peak characteristic in a wavelength region of approximately 930 nm. In addition, oxy-hemoglobin 203 and deoxy-hemoglobin 204 intersect based on an isosbestic point 210 in a wavelength region of approximately 800 nm.

According to one embodiment, the bio-signal analysis apparatus 100 includes at least four light emitting elements outputting four wavelengths and may emit light of four discrete wavelengths, which are determined based on light absorbance of water, lipid, oxy-hemoglobin, and deoxy-hemoglobin. Specifically, the discrete wavelengths emitted by each light emitting element include a first discrete wavelength adjacent to a peak region of the water 201 and a second discrete wavelength adjacent to a peak region of the lipid 202, a third discrete wavelength prior to an isoabsorption point 210 of the known absorption spectra of the oxy-hemoglobin 203 and the deoxy-hemoglobin 204, and a fourth discrete wavelength in a region adjacent to the isoabsorption point 210. At this time, the third discrete wavelength may be selected in a region where a difference in absorbance between the oxy-hemoglobin 203 and the deoxy-hemoglobin 204 is great in consideration of the absorbance of the deoxy-hemoglobin 204. For example, the first discrete wavelength may be approximately 975 nm, and the second discrete wavelength may be approximately 915 nm. In addition, the third discrete wavelength and the fourth discrete wavelength may be approximately 688 nm and approximately 808 nm, respectively, and are not limited thereto.

According to another embodiment, the bio-signal analysis apparatus 100 may include five, six, seven, or eight light emitting elements that emit additional discrete wavelengths in addition to the first to fourth discrete wavelengths described above. According to this, the fifth to eighth discrete wavelengths added accordingly may be determined according to peak characteristics of other chromophores other than the above-described chromophore (that is, water, lipid, oxy-hemoglobin, and deoxy-hemoglobin), and are not limited thereto. For example, a multi-wavelength bio-signal analysis apparatus 100 may additionally select wavelengths in a region in which inclination of the absorption spectrum of each chromophore is relatively gentle, and may also additionally select wavelengths in a predetermined interval in a main region of the absorption spectrum of each chromophore.

For example, the fifth to eighth discrete wavelengths to be added may be determined according to the peak characteristics of the absorption spectrum of collagen, melanin, methemoglobin (methHb), carbon monoxide-binding hemoglobin (CO hemoglobin (COHb)), and the like other than the above-described chromophores. Alternatively, the fifth to eighth discrete wavelengths to be added may be determined based on the center of gravity of the absorption spectrum of the above-described chromophores, for example, a wavelength in a region of approximately 700 nm and/or approximately 800 nm.

Meanwhile, although the bio-signal analysis apparatus 100 is described as including four to eight light-emitting elements in the above description, the present invention is not limited to this, and may be implemented in a form including fewer or more light-emitting elements.

In addition, the bio-signal analysis apparatus 100 may use a frequency-domain (FD) method of measuring changed amplitude and phase of a modulated light source, a bio-signal analysis method by emitting a continuous wave by using intensity of reflected light, or the like

Referring back to FIG. 2, the photodetector 120 detects output light emitted from the object 200 and converts the detected output light into an electrical signal under a control of the control unit 130. At this time, the output light emitted from the object 200 includes output light emitted through the object. The photodetector 120 may amplify AC components of the converted electrical signal. To this end, the photodetector 120 may include at least one avalanche photodiode (APD) and is not limited thereto. The photodetector 120 may be implemented in various forms such as a photodiode, a photo transistor, a photo multiplier tube (PMT), and a photo cell. In addition, the photodetector may be implemented by including a new type of optical sensor according to development of technology.

The photodetector 120 may digitize the amplified electrical signal and transmit digitized signal to the control unit 130.

In addition, the photodetectors 120 may be arranged to be spaced apart from a plurality of light emitting elements 110 at a predetermined distance to receive output light emitted from the object.

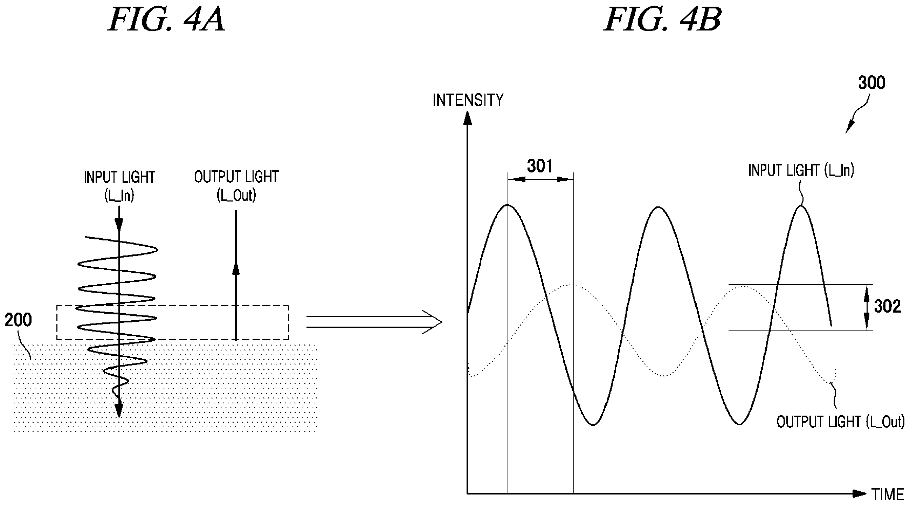

FIGS. 4A, 4B are diagrams illustrating optical characteristics of input light incident on an object by a light emitting element and output light detected by a photodetector.

FIGS. 4A, 4B exemplarily illustrate a case of an FD method, and as illustrated in FIG. 4A, if the input light of the light emitting element is applied to the object 20, the input light is scattered and absorbed by various components including chromophore in the object 20.

A graph 300 illustrated in FIG. 4B is a graph illustrating characteristics of input light L_ln and output light L-Out (that is, reflected light) in a frequency domain (FD). As the input light L_ln of the light emitting element is applied to the object 20, the reflected light L_Out detected by the photodetector 120 has characteristics of phase shift 301 and amplitude attenuation 302 with respect to the input light L_In.

The control unit 130 controls overall operations of the bio-signal analysis apparatus 100. For example, the control unit 130 may control a plurality of light emitting elements 110 and at least one photodetector 120 by executing a control program stored in a memory (not illustrated). At this time, the control unit 130 may be a processor used for a general-purpose computing device or may be implemented in the form of an embedded processor.

The control unit 130 controls drive of the plurality of light emitting elements 110 by executing the control program and calculates concentration of the chromophore in the object 20, based on the digitized electric signal received from the at least one photodetector 120, and thus, a biological configuration of the object 20 is analyzed.

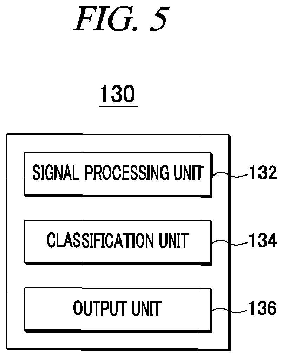

FIG. 5 is a diagram illustrating a detailed configuration of the control unit according to an embodiment of the present invention.

The control unit 130 includes configurations of a signal processing unit 132, a classification unit 134, and an output unit 136. At this time, each configuration element may be configured in the form of a software module, and functions of the signal processing unit 132, the classification unit 134, and the output unit 136 may be implemented through a memory and a processor of the control unit 130. That is, as the processor executes the bio-signal analysis program stored in the memory, the functions performed by the signal processing unit 132, the classification unit 134, and the output unit 136 are implemented.

The signal processing unit 132 controls each light emitting element 110 to output light having a previously designated wavelength, and then receives and analyzes the optical signal sensed by the photodetector 120 to extract characteristic parameters of the optical signal. For example, optical characteristics such as a wavelength of the output light emitted from the object and intensity (amplitude) or phase of the output light for each wavelength are extracted. As such, measurement values for each wavelength of the output light emitted from the object is transmitted to the classification unit 134.

The classification unit 134 is machine-learned based on training data in which the measurement values for each wavelength of the optical signal and concentration for each of a plurality of chromophore materials are matched each other based on a machine learning algorithm, and, and subsequently, if the measurement values for each wavelength of the output light emitted from the object is input, a concentration value for each chromophore material matching thereto is output.

The classification unit 134 may be learned by using a machine learning algorithm such as multi-layer perceptron or deep learning. At this time, the training data used for learning of the classification unit 134 is obtained by matching the measurement values for each wavelength of the optical signal and concentration of the plurality of chromophore materials, respectively.

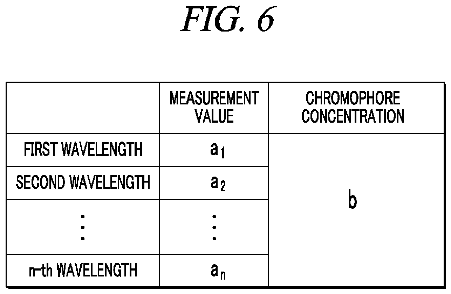

FIG. 6 is a diagram illustrating an example of the training data used for the learning of the classification unit according to an embodiment of the present invention.

As illustrated, one piece of unit training data is data in which the measurement value (for example, intensity of light) measured for each wavelength of the reflected output light and the concentration of the chromophore material matched with the measurement value are recorded. Meanwhile, although only the concentration of one chromophore material is recorded in the drawing, concentrations may be recorded by being separated from each other for each of the plurality of chromophore materials unlike this. The training data is collected according to a calculation model or other methods used for a conventional fitting algorithm, and the classification unit 134 which completes learning therethrough is mounted in each bio-signal analysis apparatus 100. The classification unit 134 may be updated by a manufacturing company or a user, and a data communication module therefor may be added to the bio-signal analysis apparatus 100.

The output unit 136 receives the concentration for each chromophore material matching the optical signal measurement value from the classification unit 134 and outputs an appropriate bio-signal analysis result by using the concentration.

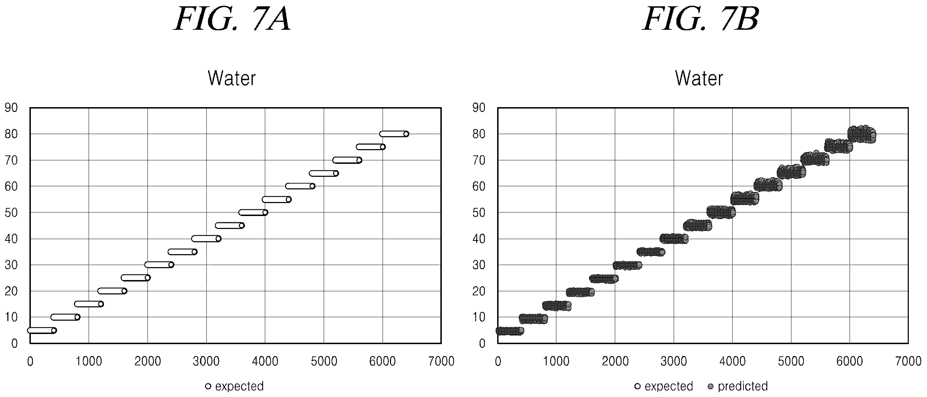

FIGS. 7A, 7B, 8A, 8B, 9A and 9B are diagrams illustrating analysis results of the bio-signal analysis apparatus according to an embodiment of the present invention.

All use simulation data generated in consideration of characteristics of the LED.

FIG. 7A illustrates an example of the training data. An X axis represents an identification number of each training data and illustrates a total of 6,400 experimental data. An Y axis represents chromophore concentration matched to each training data, and in this example, concentration of water is illustrated. The classification unit 134 may be trained by using the training data.

What is illustrated in FIG. 7B illustrates classification results when measurement values for each wavelength band of an optical signal transmitted to the signal processing unit 132 are input to the classification unit 134 learned by using the training data described above. Unlike FIG. 1B, it may be confirmed that the values classified through the classification unit 134 matches the values of the training data.

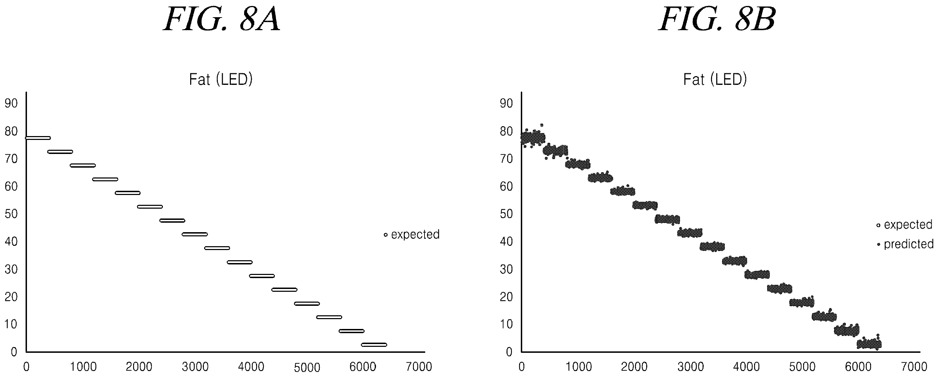

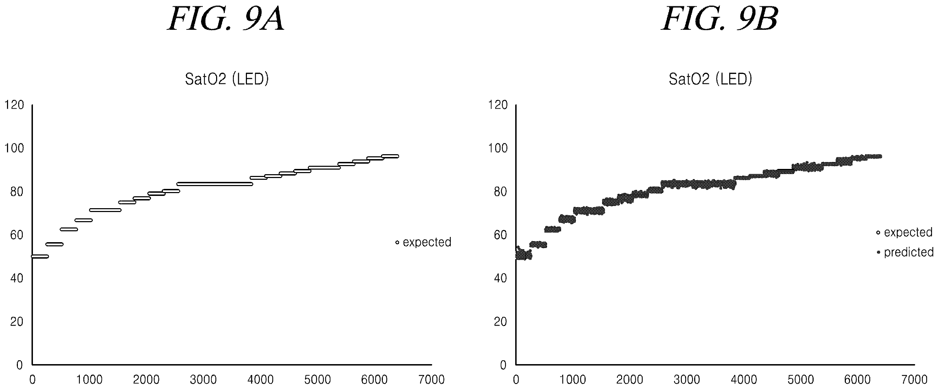

FIGS. 8A ,8B illustrate training data FIG. 8A for measuring concentration of fat in the chromophore and classification results FIG. 8B according thereto, and FIGS. 9A, 9B illustrate training data FIG. 9A for measuring concentration of hemoglobin in the chromophore and classification results FIG. 9B according thereto.

As such, it may be confirmed that even in a case where an LED is used as a light emitting element, accurate classification results are output unlike a case in which a fitting algorithm is used.

In addition, an embodiment of the present invention may be implemented in the form of a recording medium including commands executable by a computer, such as program module operated by the computer. A computer readable medium may be any available medium that is accessible by the computer and may include both volatile and nonvolatile media and removable and non-removable media. In addition, the computer readable medium may include any computer storage medium. The computer storage medium includes both volatile and nonvolatile media and removable and non-removable media implemented in any method or technology for storing information such as computer readable commands, data structures, program modules, or other data.

The above description of the present invention is for illustration only, and those skilled in the art to which the present invention belongs may understand that the present invention may be easily modified into other specific forms without changing the technical idea or essential characteristics of the present invention. Therefore, it should be understood that the embodiments described above are illustrative in all respects and are not restrictive. For example, each configuration element described as a single type may be implemented in a distributed manner, and in the same manner, configuration elements described as distributed may be implemented in a combined form.

The scope of the present invention is defined by the following claims rather than the specification described above, and it should be interpreted that all changes or modified forms derived from the meaning and scope of the claims and equivalent concepts thereof are included in the scope of the present invention.

## Claims

1. A bio-signal analysis method of a bio-signal analysis apparatus for analyzing a bio-signal based on output light emitted from an object after being output from a light source, the bio-signal analysis method comprising:
a step of providing a classification unit machine-trained based on training data in which measurement values for each wavelength of the output light and concentration for each of a plurality of chromophore materials match each other;
a step of inputting the measurement values of the output light emitted from the object measured by the bio-signal analysis apparatus into the classification unit; and
a step of outputting chromophore concentration output from the classification unit with respect to the input,
wherein the light source includes a plurality of light emitting elements for emitting output light having different wavelengths.

2. The bio-signal analysis method of claim 1, wherein
the light source includes at least one of a laser diode (LD), a light emitting diode (LED), and a vertical cavity surface emitting laser (VCSEL).

3. The bio-signal analysis method of claim 1, wherein
the training data includes measurement values of optical signals classified for each of a plurality of wavelength bands matching concentration of water, concentration of lipid, concentration of oxy-hemoglobin, and concentration of deoxy-hemoglobin corresponding to the chromophore material.

4. A bio-signal analysis apparatus, comprising:
a plurality of light emitting elements that emit output light having different wavelengths to an object;
at least one photodetector that detects the output light emitted from the object; and
a control unit that is connected to the light emitting element and the photodetector to control operations of the light emitting element and the photodetector and calculates chromophore concentration based on a measurement value of an optical signal received through the photodetector,
wherein the control unit includes a classification unit machine-trained based on training data in which measurement values for each wavelength of the output light and concentration for each of a plurality of chromophore materials match each other, and inputs the measurement values of the output light emitted from the object measured by the photodetector to the classification unit to calculate the chromophore concentration.

5. The bio-signal analysis apparatus of claim 4, wherein
the light source includes at least one of a laser diode (LD), a light emitting diode (LED), and a vertical cavity surface emitting laser (VCSEL).

6. The bio-signal analysis apparatus of claim 4, wherein
the training data includes measurement values of optical signals classified for each of a plurality of wavelength bands matching concentration of water, concentration of lipid, concentration of oxy-hemoglobin, and concentration of deoxy-hemoglobin corresponding to the chromophore material.

7. The bio-signal analysis apparatus of claim 4, wherein
the control unit sequentially drives each light emitting element such that the plurality of light emitting elements each emit the output light.
